# EUROPEAN PATENT SPECIFICATION

(11) **EP 1 112 695 B1**
(45) Date of publication and mention of the grant of the patent: **13.02.2008**
(21) Application number: 99923370.3
(22) Date of filing: 03.06.1999
(51) Int. Cl.: A41B 9/02, A61F 5/40

(54) **A KIND OF MAN'S BRIEFS AND LINING OF THE BRIEFS**
HERRENUNTERHOSE UND IHR FUTTER
SLIP POUR HOMME ET DOUBLURE ASSOCIEE

(30) Priority: 11.06.1998 CN 98205905
(43) Date of publication of application: 04.07.2001
(73) Proprietor: Qi, Xiaoming, Chao Yang District, Beijing 100013 (CN)
(72) Inventor: Qi, Xiaoming, Chao Yang District, Beijing 100013 (CN)
(74) Representative: Reinhard - Skuhra - Weise & Partner
(86) International application number: PCT/CN1999/000075
(87) International publication number: WO 1999/063845

(56) References cited:
- US-A- 3 499 442
- US-A- 4 471 772
- US-A- 5 524 298

## Description

### Field of the Invention

This invention relates to protective underwear, specifically to briefs for men and lining for the briefs.

### Background of the Invention

It has been proved by medical science and practice that men's testicles will drop down as the scrotum becomes loosened. While the testicles drop to a certain extent, they will be apt to be pressed and pushed by the upper ends of the legs. Especially, when the man is at a sitting posture, this problem will not only make him uncomfortable, but also affect his health.

Existing men's briefs are designed mainly on the basis of their comfort such as softness, lightness, and performance of sweat absorption etc. while being worn. Therefore, most of them are based on a selection of materials, that is, comfortable materials are adopted to make the briefs. However, even though the briefs are made from rather comfortable materials, whether in triangular shape or in rectangular shape, they do not have the function to support and hold the scrotum above the upper ends of the legs, and therefore cannot solve the above-mentioned problem.

A man's supporter brief undergarment is already known from document US 3,499,442 which includes an elastic U-shaped pouch strap attached to an elastic waistband, and an elastic panel attached to and located inside the U-shaped strap to provide a shaped, supporting pouch at the front of the garment. A pair of elastic leg straps are attached to the U-shaped strap substantially at the strap bight, and are attached to opposite sides of the U-shaped strap to provide leg openings. A cloth covering is attached to and extends between the waistband, pouch strap and leg straps.

### Summary of the Invention

The object of the present invention is to provide briefs for men and lining of the briefs, which can control the extent of dropping of the scrotum and support and hold them above the upper ends of the legs, thereby preventing the testicles from being pressed and pushed.

The invention is based on a support belt, which is added to the front section of the men's briefs and lining. The bottom of the support belt has an arc shape and forms a bag together with the bottom portion of the front section, i.e. the portion between the bottom of the tightening up section of the scrotum and the upper ends of the legs. Both ends of the support belt have a flared shape and are diverging upwardly, and are connected with the front section and the waist section to facilitate the stability of the support belt and the support force. The support belt can be adjusted easily. It can be adjusted by changing the width of the flared mouth of the support belt, the length of the support belt or the tension of the support belt, so as to obtain a proper support position and a comfortable support force.

When the men's briefs and lining are put on, the bottom tightening section of the support belt is positioned between the bottom of the tightening portion of the scrotum and the groin above the upper ends of the legs by adjusting the support belt, thereby preventing the testicles from dropping. While the support belt avoids the dropping of the scrotum, it also offers a support function together with the bag, which is formed by the support belt and the front section. The structure combination of the support belt, the front section and the waist section can effectively control the extent of dropping of the scrotum and prevent the testicles from being pressed by the upper ends of the legs.

The present invention has the following advantages: As only one support belt is added to the front section of the men's briefs and lining, its structure is very simple, since the dropping of the scrotum is restrained by the support belt. The performance of breathability, ventilation and temperature reduction in the environment of the upper ends of the legs is improved significantly. The process of putting the briefs on is very convenient, since the support belt and the front section are connected with each other. Moreover, the support belt has no special requirements on material, since the present invention is targeted toward the support function.

### Descriptions of the attached drawings

- Figure 1: illustrates the structure of the triangular briefs and lining proposed according to the invention.
- Figure 2: is a side view of the triangular briefs and lining according to the invention while being worn.
- Figure 3: illustrates the structure of the rectangular briefs and lining proposed according to the invention.
- Figure 4: is a side view of the rectangular briefs and lining according to the invention while being worn.
- Figure 5: illustrates the structure of a second embodiment of the invention.
- Figure 6: illustrates the structure of a third embodiment of the invention.
- Figure 7: illustrates the structure of a fourth embodiment of the invention.
- Figure 8: illustrates the structure of a fifth embodiment of the invention.
- Figure 9: illustrates the structure of a sixth embodiment of the invention.
- Figure 10: illustrates another structure of a sixth embodiment of the invention.

### Detailed description of the Embodiments

### Embodiment 1

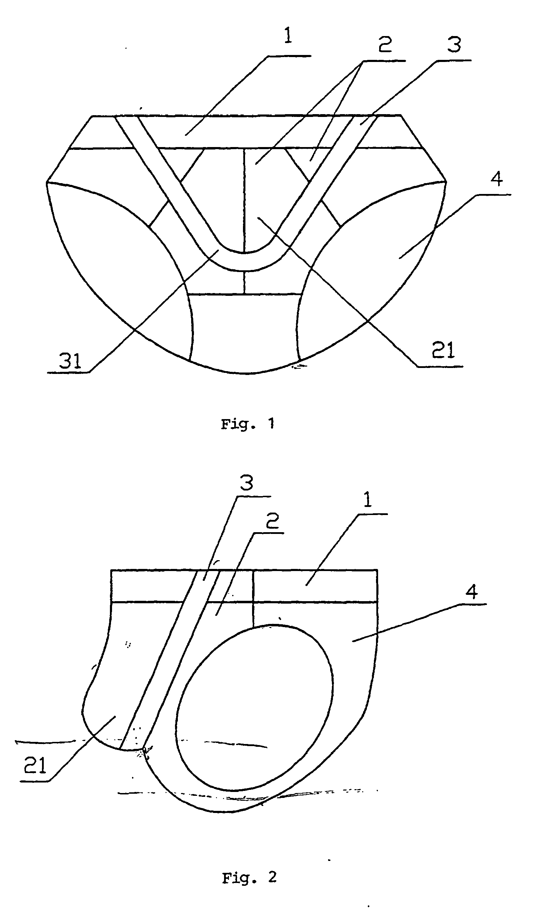

As shown in Figures 1 and 2, the present invention comprises a waist section 1, a front section 2, a back section 4, and a support belt 3 which is connected with the front section 2. The support belt 3 is a belt-like section connected with the front section 2. Both ends of the support belt 3 have a flared shape and are diverging upwardly and are connected with the front section 2 and the waist section 1 of the front section so as to facilitate the stability of the support belt 3. The bottom of the support belt has an arc shape, and forms a bag together with the bottom portion of the front section 2 to facilitate the supporting and holding functions.

As the support belt 3 according to the present invention needs stability of connection, different means of connection can be used in accordance with the different materials of the briefs. In addition, the support belt should be adjusted according to various requirements on the supporting force and supporting position.

For example, in the structure shown in this embodiment 1, the front section 2 of the briefs is made of a kind of popular and soft knitted cloth. The support belt 3 is made of low elasticity material. The entire support belt 3 is fixed on the front section 2 and the waist section 1, and a bag section 21 is formed by elasticity of the material of the front section 2 surrounded by the arc section 31 of the bottom of the support belt 3. The bottom of the bag section 21, which is surrounded by the support belt 3, should be located between the bottom of the tightening position of the scrotum and the upper ends of the legs. With the above-mentioned structure, when the briefs are worn, the bottom of the support belt is right between the bottom of the tightening position of the scrotum and the groin above the upper ends of the legs, thus forming an obstacle against dropping of the scrotum. While avoiding dropping of the scrotum, the support belt together with the bag formed by the support belt and the front section also offer a support and holding function for the scrotum.

With the above-mentioned connection means, the support belt 3 can also be made of elastic materials, and a bag section 21 is formed by the bottom of the front section 2 and elasticity of the support belt 3 at the position connected with the front section 2 and the waist section 1 along the entire length of the support belt 3. Or, when the support belt 3 is connected with the front section 2 and the waist section 1 along the entire length of the support belt 3, an elastic belt 31 can be added on the support belt 3 between the front section 2 and the waist section 1.

The above-mentioned connection means for fixing the entire length of the support belt 3 can be designed to be different specifications according to different bodies so as to meet various requirements.

Figure 2 is an illustration of the first embodiment of the present invention while being worn. When it is worn, the tightening portion of the bottom of the support belt 3 is between the bottom of the tightening position of the scrotum and the groin above the upper ends of the legs. Therefore, it prevents dropping of the scrotum. While preventing the dropping of the scrotum, the support belt 3 together with the bag 21 formed by the support belt 3 and the front section 2 also hold the scrotum. Since the support belt 3 is adjustable, the structure of the support belt 3 can control the extent of dropping of the scrotum, and thus secures the testicles against being pressed by the upper ends of the legs.

With the above-mentioned description, since only a support belt 3 is added to the front section 2 of the men's briefs and lining, the structure of this invention is very simple and will not affect comfort while the briefs are worn. Meanwhile, since the structure of the support belt 3 can effectively restrain dropping of the scrotum, the performance of breathability, ventilation and temperature reduction in the environment of the upper ends of the legs is improved significantly. Since the support belt 3 and the front section 2 are connected with each other, the process of putting on is very convenient. Moreover, because this invention is targeted toward a support function, the support belt has no special requirements on material.

### Embodiment 2

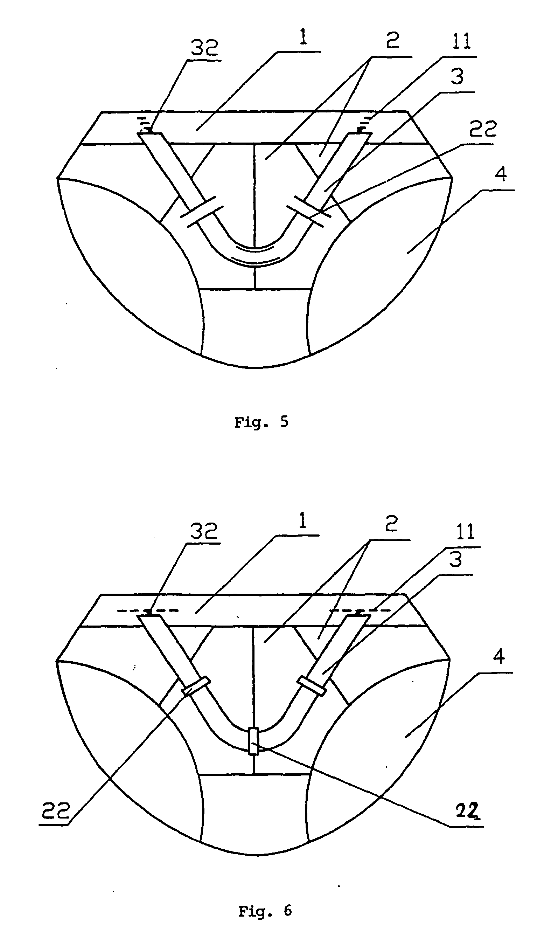

The second embodiment of the present invention is shown in Figure 5. In this embodiment, the support belt 3 is connected to the front section 2 and the waist section 1 only at three points, and the arc section 31 at the bottom thereof is fixed and connected to the extreme bottom of the bag section of the front section 2 so as to ensure stability of the bottom.section of the support belt 3. Its top flared mouth is connected to the waist section 1. In order to make the supporting position of the support belt 3 comfortable, the support belt 3 should be adjusted when the briefs are put on. A length adjustment device can be installed on the support belt 3, and the supporting position of the support belt 3 can be controlled by adjusting the length of the support belt. Various existing length adjustment methods can be utilized in connection with the length adjustment device. For example, several hooks 32 are installed at either end of the support belt 3, and several holes 11 are provided in the longitudinal direction of the waist section 1. The length of the support belt can be adjusted by hooking up the hook 32 at holes of different height so as to adjust the supporting force. In addition, other popular length adjustment devices such as taps, nylon tabs and buttons etc. can also be used in the length adjustment device of this invention. The support belt 3 can also be made of two segments, with several holes being made on one segment, while several hooks are installed on the corresponding segment.

In this embodiment, since the support belt 3 is connected to the front section 2 and the waist section 1 at three points, a positioning device is installed between the top and bottom connection points of the support belt 3 so as to stabilize the portion of the support belt 3 between the top and bottom connection points on the front section 2. The positioning device can be a hole 22 on the front section 2, allowing the support belt 3 to penetrate through the hole 22 and form a stable integration with the front section 2. The positioning device can also be a tab installed on the front section, and the width of the mouth of the tab is slightly greater than the width of the support belt 3 so as to allow the support belt 3 to have a little position adjustment function in the transverse direction.

With the above mentioned structure, this embodiment has a good adjustment function. At the beginning of use, the user can make some pre-adjustments according to various requirements, and no further adjustments will be needed after it has been adjusted to be comfortable. The structure of this embodiment has better adaptability and will better suit various needs of the users.

### Embodiment 3

This embodiment is shown in Figure 6, which is basically the same as embodiment 2. The support belt 3 is connected to the front section 2 and the waist section 1 of the briefs at three points. The difference is that the arc section 31 of the bottom of the support belt 3 is connected to the bottom of the bag section of the front section 2 via the tab device 22 so as to ensure stability of the bottom of the support belt 3, and the top flared mouth of the support belt 3 is connected to the waist section 1, and the support belt 3 should be adjusted while putting on the briefs so as to make the supporting position of the support belt 3 comfortable. To adjust the width of the flared mouth at the top of the support belt 3, in this embodiment, several holes 11 can be made horizontally along the waist section 1, and several hooks 32 can be installed at the flared mouth end of the support belt 3. The width of the flared mouth can be adjusted by changing the hooking position of the hook 32. When the width of the mouth is great, the arc section at the bottom of the support belt 3 will be lifted up, whereas when the width of mouth is small, the arc section at the bottom of the support belt 3 will be lowered, thereby realizing adjustments of high or low positions of the support belt 3. Buttons at different positions of the waist section or other fast connection structures can also be used to adjust the width of the mouth end.

### Embodiment 4

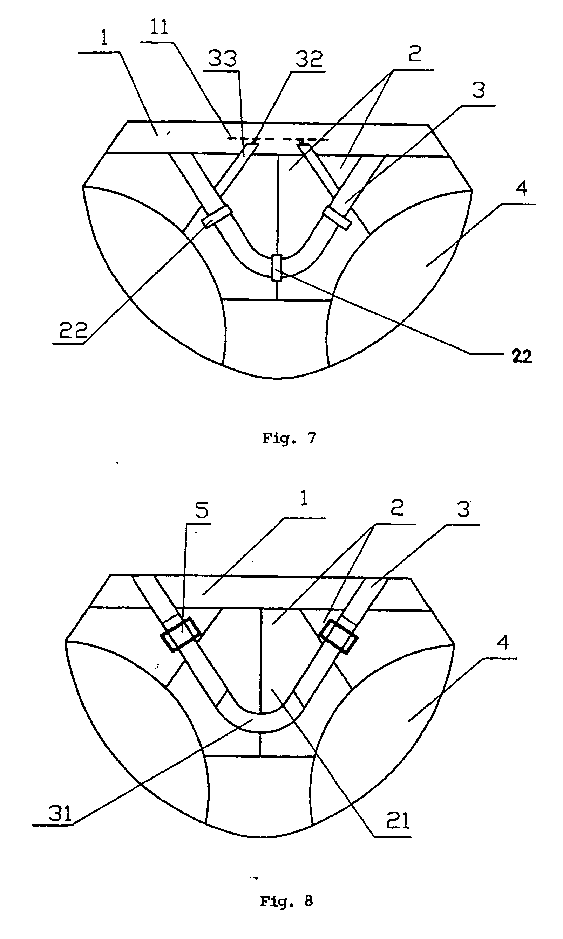

In the fourth embodiment of this invention shown in Figure 7, the structure of this embodiment is identical to that of embodiment 3. The width of the flared mouth at the top of the support belt 3 is adjusted by an auxiliary belt 33 fixed on the support belt 3. Pulling inwardly or loosening the support belt by the auxiliary belt can change the width of the mouth of the support belt 3.

### Embodiment 5

As shown in Figure 8, in this embodiment, only the arc section 31 at the bottom of the support belt 3 is made of elastic materials such as elastic ribbons, etc. The arc section 31 can be connected to the bottom of the front section 2, or connected to the bottom of the front section 2 with its bottom. With the elasticity of the arc section 31, a bag section 21 can be formed by tightening the arc section 31 at the bottom of the front section 2. Various elastic structures of the support belt 3 described before offer the capability to adjust the supporting force of the support belt 3, and thus control the extent of dropping of the scrotum.

The support belt 3 can also be connected tightly to the front section 2 and the waist section 1 with its top diverging section. Its bottom section is loosened, and a length adjustment device 5 is installed at a proper position of the loosened section. Existing various length adjustment devices are all suitable for this invention. The function of the length adjustment device 5 is to adjust the supporting force according to the user's special condition, so as to control the extent of dropping of the scrotum.

With the above description, this invention can fulfill various means of connections and adjustments, and its aim is to make users with different demands feel more comfortable while wearing this kind of men's briefs. Various connection methods and adjustment methods of the supporting force mentioned above are only used to describe this invention, and do not limit the present invention.

### Embodiment 6

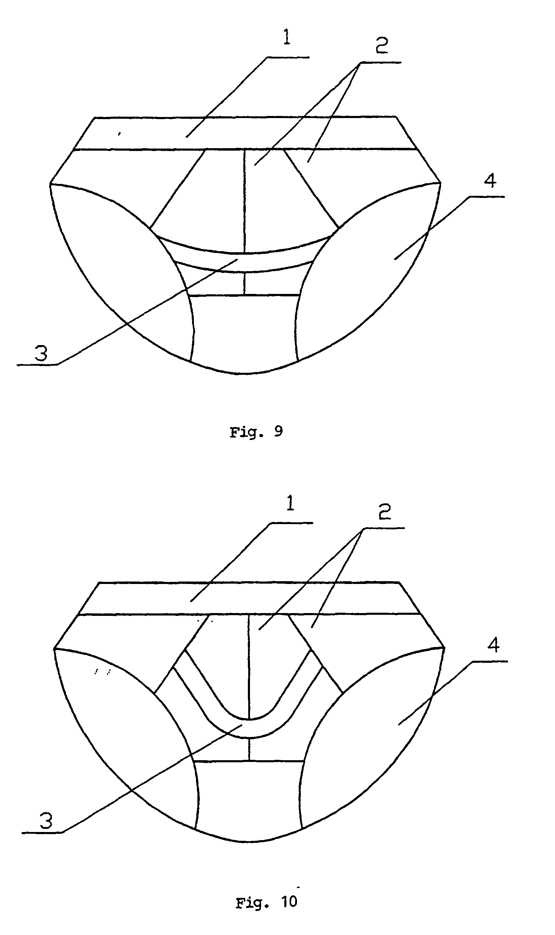

This embodiment is shown in Figure 9. The support belt 3 has an arc shape and is located between the two trousers legs at the bottom of the front section 2 and is fixed. It can be designed to be different specifications. The support belt 3 can also be fixed at the joint of slanted stitches of the front section 2, as shown in Figure 10, and this embodiment is designed based on a kind of popular triangular briefs. In the above mentioned embodiment, if the front section 2 of the triangular briefs has a double-layer structure (such double-layer structure is popular and is not shown in the figure), the support belt 3 can be passed through the mouth of the double-layer front section into the double-layer structure after its bottom is fixed, and then the above mentioned various connections, positioning and adjustment devices can be connected into the interlayer of the double-layer structure of the front section 2. This kind of structure can eliminate discomfort of the user due to a thick front section while the support belt 3 has a supporting function.

### Embodiment 7

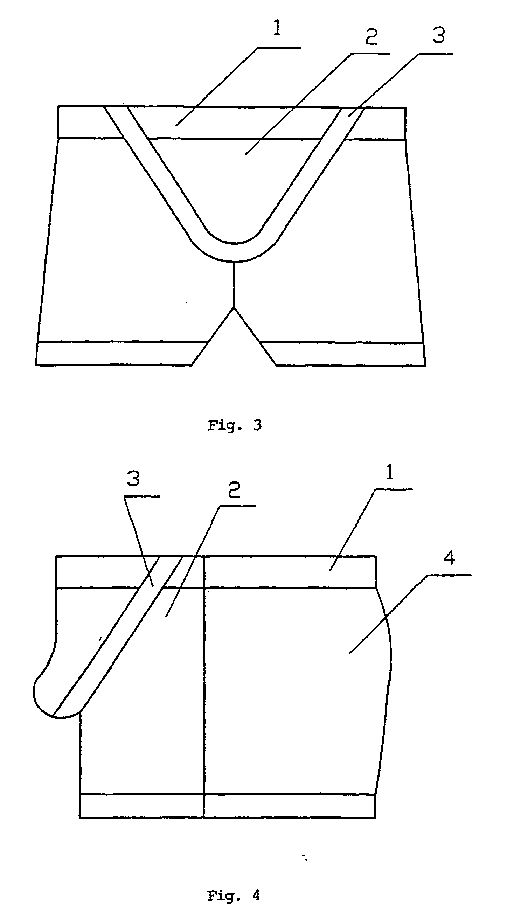

As shown in Figures 3 and 4, in this embodiment, the support belt 3 is connected to the front section 2 and the waist section 1 of a kind of right-angled men's briefs and lining. A bag section 21 is formed by the support belt 3 and the portion of the front section 2 which is surrounded by it. The connection method is the same as that in the first embodiment and is not stated again. The support function of this embodiment is entirely identical to that of the above statement.

## Claims

1. A kind of man's briefs and lining of the briefs, comprising a waist section (1), a front section (2) and a back section (4)
**characterised in that**,
a support belt (3) is connected to the front section (2) and both ends of the support belt (3) are connected to the waist section (1) and an arc section (31) of the bottom of the support belt (3) is disposed right between the bottom of the tightening position of the scrotum and the groin above the upper ends of the legs, thus forming an obstacle against dropping of the scrotum.

2. A kind of man's briefs and lining of the briefs as described in claim 1,
**characterised in that**,
a bag section (21) is formed by the support belt (3) and the front section (2).

3. A kind of man's briefs and lining of the briefs as described in claim 1,
**characterised in that**,
both ends of the support belt (3) are flared up.

4. A kind of man's briefs and lining of the briefs as described in claim 1,
**characterised in that**,
the length of the support belt (3) is adjustable.

5. A kind of man's briefs and lining of the briefs as described in claims 1 or 3,
**characterised in that**,
the width of the flared mouth of the support belt (3) is adjustable.

6. A kind of man's briefs and lining of the briefs as described in claims 1 or 4,
**characterised in that**,
the support belt (3) has an elastic structure so as to adjust the supporting force.

7. A kind of man's briefs and lining of the briefs as described in claims 1 or 4,
**characterised in that,**
a length adjustment device (5) is installed on the support belt (3) so as to adjust the supporting force.

## Patentansprüche

1. Herrenunterhose und Futter für Unterhosen, welche aufweisen: einen Taillenabschnitt (1), einen Vorderabschnitt (2) und einen rückseitigen Abschnitt (4),
**dadurch gekennzeichnet, dass**
ein Stützgurt (3) mit dem Vorderabschnitt (2) verbunden ist und beide Enden des Stützgurtes (3) mit dem Taillenabschnitt (1) verbunden sind und ein Bogenabschnitt (31) am untersten Teil des Stützgurtes (3) genau zwischen dem untersten Teil der Befestigungsposition des Skrotums und der Leiste oberhalb der oberen Enden der Beine angeordnet ist, so dass dadurch ein Hindernis gegen das Herabfallen des Skrotums gebildet ist.

2. Herrenunterhose und Futter der Unterhose nach Anspruch 1,
**dadurch gekennzeichnet, dass**
ein Beutelabschnitt (21) durch den Stützgurt (3) und den Vorderabschnitt (2) gebildet ist.

3. Herrenunterhose und Futter der Unterhose nach Anspruch 1,
**dadurch gekennzeichnet, dass**
beide Enden des Stützgurtes (3) glockenförmig sind.

4. Herrenunterhose und Futter der Unterhose nach Anspruch 1,
**dadurch gekennzeichnet, dass**
die Länge des Stützgurtes (3) einstellbar ist.

5. Herrenunterhose und Futter der Unterhose nach Anspruch 1 oder 3,
**dadurch gekennzeichnet, dass**
die Breite der glockenförmigen Öffnung des Stützgurtes (3) einstellbar ist.

6. Herrenunterhose und Futter der Unterhose nach Anspruch 1 oder 4,
**dadurch gekennzeichnet, dass**
der Stützgurt (3) eine elastische Struktur besitzt, um so die Stützkraft einzustellen.

7. Herrenunterhose und Futter der Unterhose nach Anspruch 4,
**dadurch gekennzeichnet, dass**
eine Längeneinstellvorrichtung (5) an dem Stützgurt (3) angebracht ist, um so die Stützkraft einzustellen.

## Revendications

1. Type de slip pour homme et de doublure pour celui-ci, comprenant une partie de ceinture (1), une partie avant (2) et une partie arrière (4),
**caractérisé en ce que**
une bande de soutien (3) est connectée à la partie avant (2) et que les extrémités de la bande de soutien (3) sont connectées à la partie de ceinture (1) et qu'une partie en arc (31) de la partie inférieure de la bande de soutien (3) est disposée exactement entre le bas de la position de resserrement du scrotum et l'aine au dessus des extrémités des jambes, en formant de la sorte un obstacle contre la retombée du scrotum.

2. Type de slip pour homme et de doublure pour celui-ci comme décrit dans la revendication 1,
**caractérisé en ce que**
une partie de poche (21) est formée par la bande de soutien (3) et la partie avant (2).

3. Type de slip pour homme et de doublure pour celui-ci comme décrit dans la revendication 1,
**caractérisé en ce que**
les deux extrémités de la bande de soutien (3) sont évasées.

4. Type de slip pour homme et de doublure pour celui-ci comme décrit dans la revendication 1,
**caractérisé en ce que**
la longueur de la bande de soutien (3) est réglable.

5. Type de slip pour homme et de doublure pour celui-ci comme décrit dans la revendication 1,
**caractérisé en ce que**
la largeur de l'embouchure de la bande de soutien (3) est réglable.

6. Type de slip pour homme et de doublure pour celui-ci comme décrit dans la revendication 1,
**caractérisé en ce que**
la bande de soutien (3) présente une structure élastique de façon à adapter la force de soutien.

7. Type de slip pour homme et de doublure pour celui-ci comme décrit dans la revendication 1,
**caractérisé en ce que**
un dispositif de réglage de longueur (5) est installé sur la bande de soutien (3) de façon à adapter la force de soutien.
